# FASCICULE DE BREVET EUROPEEN

(11) **EP 3 094 368 B1**
(45) Date de publication et mention de la délivrance du brevet: **21.03.2018**
(21) Numéro de dépôt: 15700573.7
(22) Date de dépôt: 14.01.2015
(51) Int. Cl.: A61M 37/00, A61N 1/32, A61N 1/04

(54) **DISPOSITIF POUR L'ÉLECTROSTIMULATION ET/OU L'IONTOPHORÈSE**
VORRICHTUNG ZUR ELEKTROSTIMULATION UND/ODER IONTOPHORESE
DEVICE FOR ELECTROSTIMULATION AND/OR IONTOPHORESIS

(30) Priorité: 14.01.2014 FR 1450258
(43) Date de publication de la demande: 23.11.2016
(73) Titulaire: Feeligreen, 06130 Grasse (FR)
(72) Inventeur: BIANCHI, Christophe, 06100 Nice (FR)
(74) Mandataire: Hautier, Nicolas
(86) Numéro de dépôt international: PCT/EP2015/050614
(87) Numéro de publication internationale: WO 2015/107090

(56) Documents cités:
- EP-A2- 0 964 722
- FR-A1- 2 778 108
- US-A1- 2002 038 101
- US-A1- 2007 093 743

## Description

### DOMAINE TECHNIQUE DE L'INVENTION

La présente invention concerne un dispositif pour l'électrostimulation et/ou l'iontophorèse.

L'invention concerne les techniques consistant à soumettre des cellules dermiques à un champ électrique pour augmenter leur activité et/ou à utiliser un courant électrique pour faciliter la diffusion transdermique de substances actives, et concerne plus particulièrement un dispositif d'électrostimulation et/ou iontophorèse.

La stimulation cellulaire par un champ électrique ou électrostimulation est utilisée dans de nombreuses applications. Elle est connue pour diminuer la douleur ou bien pour des applications cosmétiques comme le traitement des rides. On soumet par exemple des fibroblastes à un champ électrique pour augmenter leur taux de production de protéine. Les protéines produites par les fibroblastes étant essentiellement le collagène et l'élastine, l'effet attendu est une amélioration de l'élasticité de la peau et donc un effet visible de réduction des rides. L'électrostimulation peut aussi être utilisée pour le traitement de la cicatrisation pour laquelle le champ électrique augmente la migration cellulaire et facilite ainsi la cicatrisation.

L'iontophorèse quant à elle est une technique qui peut être utilisée à des fins cosmétiques et/ou médicales pour introduire des substances actives dans le derme de la peau au moyen d'un courant.

### ETAT DE LA TECHNIQUE

Contrairement aux modes d'administration traditionnels par voie orale ou parentérale, la durée d'application du courant pour l'iontophorèse ou l'électrostimulation est longue. La source de courant doit donc être adaptée pour fournir un courant pendant une longue période. En outre, ces dispositifs nécessitent également des moyens de contrôle du courant fourni, par exemple sous forme d'un dispositif électronique pouvant être programmé en fonction du traitement ou de l'effet recherché. Le dispositif électronique et la source de courant sont associés à l'élément destiné à être en contact avec la peau. Ces dispositifs sont donc souvent imposants par leur taille avec une source de courant et un dispositif électronique encombrants.

Ces dispositifs sont chers et sont ainsi réutilisés pour plusieurs patients, les éléments étant en contact avec la peau devant alors être nettoyés voir stérilisés pour assurer les règles de sécurité sanitaire. Par ailleurs, dans le cadre d'une utilisation iontophorètique, il est souvent difficile de modifier l'actif à administrer. On se retrouve alors avec un dispositif complet pour chaque actif à administrer.

Il existe donc le besoin de proposer un dispositif qui permette une utilisation individuelle voire à usage unique tout en limitant les coûts.

### RESUME DE L'INVENTION

A cet effet la présente invention concerne un dispositif d'électrostimulation ou d'iontophorèse comprenant un support comprenant une première face destinée à être en regard de la peau et une deuxième face opposée à la première. La première face comprend des électrodes, un circuit électrique reliant les électrodes et des moyens de connexion reliant le circuit électrique à un module électronique. Le dispositif comprend des moyens de détachement partiel flexibles portant au moins une partie des moyens de connexion de sorte que les moyens de connexion puissent être déportés en dehors de la première face du support, préférentiellement au niveau ou au-delà de la deuxième face du support.

Le dispositif selon l'invention permet une facilité de fabrication en formant l'ensemble des éléments électriques conducteurs sur une seule face du support. L'invention permet également un assemblage simplifié puisque la connexion électrique des électrodes au module électronique s'obtient en fléchissant les moyens de détachement partiel jusqu'à atteindre le module électronique disposé sur la deuxième face du support.

De manière avantageuse, les moyens de détachement partiel forment au moins une languette portant les moyens de connexion. Préférentiellement, le module électronique est agencé entre la au moins une languette déportée au niveau ou au-delà de a deuxième face du support et ladite deuxième face du support. Cette disposition présente l'avantage d'un assemblage aisé et sécurisé du module électronique sur le support.

Suivant un autre aspect, l'invention concerne un procédé de fabrication d'un dispositif d'électrostimulation et/ou d'iontophorèse tel que décrit ci-dessus comprenant les étapes suivantes : réalisation par dépôt d'au moins une couche de matériau conducteur sur une première face d'un support d'électrodes, d'un circuit électrique reliant les électrodes et de moyens de connexion, avantageusement dépôt d'une couche d'un matériau isolant sur le circuit électrique de sorte à l'isoler électriquement de la peau sur laquelle le dispositif est destiné à être appliqué, formation des moyens de détachement partiel des moyens de connexion. Préférentiellement, formation de découpe dans le support pour former des moyens de détachement partiel des moyens de connexion. Préférentiellement, connexion d'un module électronique aux moyens de connexion au niveau de la deuxième face du support.

Suivant un autre aspect, l'invention concerne un procédé d'assemblage du support et du module électronique d'un dispositif tel que décrit ci-dessus par la connexion de moyens de détachement partiel pour amener les moyens de connexion au niveau ou au-delà de la deuxième face du support.

Suivant un autre aspect indépendant, l'invention concerne un dispositif pour l'électrostimulation et/ou iontophorèse comprenant un support présentant une première face destiné à être placé au regard de la peau et une deuxième face opposée à la première, un module électronique disposé sur la deuxième face du support, caractérisé par le fait que : le support comprend des électrodes sur la première face, des moyens de connexion électriques et physiques destinés à connecter et à fixer le module électronique sur la deuxième face du support, et un circuit électrique reliant les électrodes et les moyens de connexion, le support est une surface souple supérieure à celle du module électronique configurée pour se conformer aux zones d'application sur le corps humain sur lesquelles elle est destinée à s'appliquer.

Le procédé de fabrication est donc particulièrement simple et avantageusement biocompatible.

### BREVES DESCRIPTION DES FIGURES

Les dessins sont donnés à titre d'exemples et ne sont pas limitatifs de l'invention. Ils constituent des représentations schématiques de principe destinées à faciliter la compréhension de l'invention et ne sont pas nécessairement à l'échelle des applications pratiques.
La figure 1 représente le support du dispositif selon l'invention vue de la première face.
La figure 2 représente le support du dispositif selon l'invention vue de la deuxième face avec le module électronique connecté. Le circuit électrique, les électrodes, et les moyens de connexions sont présentés en traits pleins pour plus de clarté. Toutefois, ils sont agencés sur la première face comme décrit ci-après.
La figure 3 représente une vue en coupe du dispositif avec le module électronique assemblé sur le support.

### DESCRIPTION DETAILLEE DE L'INVENTION

Avant d'entamer une revue détaillée de modes de réalisation de l'invention, sont énoncées ci-après des caractéristiques optionnelles qui peuvent éventuellement être utilisées en association ou alternativement.

On rappelle tout d'abord que l'invention porte sur un dispositif pour l'électrostimulation et/ou l'iontophorèse comprenant un support présentant une première face destinée à être placée au regard de la peau et portant des électrodes configurées pour être au contact de la peau et une deuxième face opposée à la première sur laquelle est destiné à être disposé un module électronique avantageusement configuré pour fournir un courant aux électrodes et/ou pour contrôler un courant fourni aux électrodes caractérisé par le fait que le support comprend sur sa première face un circuit électrique reliant les électrodes et des moyens de connexion destinés à connecter le circuit électrique au module électronique et que le dispositif comprend des moyens de détachement partiel flexibles portant au moins une partie des moyens de connexion et configurés pour permettre de rabattre par flexion lesdits moyens de connexions au-delà de la première face vers la deuxième face préférentiellement jusqu'à atteindre le module électronique

Avantageusement, les moyens de détachement partiel forment au moins une languette portant les moyens de connexion de sorte qu'une flexion de la au moins une languette amène lesdits moyens de connexion au niveau de la deuxième face ou au-delà pour connecter le module électronique.

Avantageusement, le dispositif comprend le module électronique est configuré pour être séparable du support.

Avantageusement, le dispositif comprend le module électronique est configuré pour être amovible du support.

Avantageusement, le module électronique est en appui sur la deuxième face du support et est disposé entre ladite deuxième face du support et la au moins une languette.

Avantageusement, les moyens de détachement partiel forment au moins deux languettes.

Avantageusement, les deux languettes rabattues par flexion au-delà de la première face au niveau ou au-delà de la deuxième face pour connecter les moyens de connexion au module électronique exercent une force d'appui sur ledit module électronique configurée pour plaquer ledit module électronique sur la deuxième face du support.

Avantageusement, le module électronique est entièrement soutenu par la deuxième face du support.

Avantageusement, les moyens de connexion et le module électronique sont configurés pour coopérer de manière à ce que leur coopération assure simultanément une connexion électrique du module électronique au circuit électrique et un maintien physique du module électronique sur la deuxième face du support.

Avantageusement, les moyens de connexion et le module électronique sont configurés pour coopérer de manière à ce que leur coopération assure à elle seule simultanément une connexion électrique du module électronique au circuit électrique et un maintien physique du module électronique sur la deuxième face du support.

Avantageusement, le maintien physique du module électronique est uniquement assuré par les moyens de connexion et le support.

Avantageusement, le support est une nappe souple de surface supérieure à celle du module électronique configurée pour se conformer aux zones d'application sur le corps humain sur lesquelles elle est destinée à être appliqué.

Avantageusement, la surface du support est au moins cinq fois supérieure à celle du module électronique.

Avantageusement, le support est sécable de sorte à être adaptable à la surface sur laquelle il est destiné à être appliqué.

Avantageusement, le dispositif comprend un film de matériau isolant électriquement destiné à isoler électriquement ledit circuit électrique de la peau sur laquelle le dispositif est destiné à être appliqué.

Avantageusement, le circuit électrique, les électrodes et les moyens de connexions sont formés par une encre conductrice imprimée ou sérigraphiée sur la première face du support.

Avantageusement, le support comprend au moins un élément actif cosmétique ou thérapeutique disposé sur sa première face.

Avantageusement, le support comprend une trame en biocellulose imbibé sur la première face du support d'au moins un élément actif.

Avantageusement, le dispositif comprend une trame textile étant recouverte sur la première face du support d'un hydrogel contenant au moins un élément actif.

Avantageusement, le support comprend un adhésif sur sa première face de sorte à maintenir le dispositif appliqué sur la peau.

Avantageusement, le module électronique comprend une source de courant autonome.

Avantageusement, le support est à usage unique.

Suivant un autre aspect, l'invention porte sur un procédé de fabrication d'un dispositif d'électrostimulation et/ou d'iontophorèse tel que décrit précédemment comprenant les étapes suivantes : réalisation par dépôt d'au moins une couche de matériau conducteur sur une première face d'un support d'électrodes, d'un circuit imprimé reliant les électrodes et de moyens de connexion, dépôt d'une couche d'un matériau isolant sur le circuit imprimé de sorte à l'isoler électriquement de la peau sur laquelle le dispositif est destiné à être appliqué, formation par découpe des moyens de détachement partiel des moyens de connexion.

Suivant un autre aspect, l'invention porte un procédé d'assemblage d'un dispositif tel que décrit précédemment comprenant la flexion des moyens de détachement partiel de sorte à amener les moyens de connexion au niveau ou delà de la deuxième face du support et la connexion desdits moyens de connexion avec le module électronique.

L'invention concerne un dispositif configuré pour l'électrostimulation et/ou l'iontophorèse. Le dispositif comprend un support 1 comprenant une première face 2a et une deuxième face 2b opposée. Préférentiellement, le dispositif comprend un module électronique 3.

Le support 1 est destiné à être appliqué sur la peau par sa première face 2a. Le support 1 comprend des électrodes 4 sur la première face 2a de sorte à être au contact de la peau. Ces électrodes 4 permettent de transmettre un courant électrique à la peau. Les électrodes 4 sont avantageusement affleurant à la surface de la première face 2a. Selon une autre possibilité, les électrodes sont en reliefs ou en creux au niveau de la première face 2a.

On entend par « en contact », un contact direct ou indirect. Dans le cas d'iontophorèse, la première face 2a du support 1 n'est pas directement appliquée sur la peau, le contact est indirect, par exemple par l'intermédiaire d'une couche contenant des éléments actifs. Des exemples de couches et d'éléments actifs sont donnés plus avant dans la description. Si le dispositif est utilisé comme dispositif d'électrostimulation, la première face 2a du support 1 est appliquée directement sur la peau de l'utilisateur sans couche intermédiaire contenant des éléments actifs.

Le dispositif selon l'invention est maintenu en contact avec la peau par des moyens de fixation. Les moyens de fixation sont par exemple des rubans ou fils pouvant être élastiques et enserrant la zone d'application. A titre préféré, les moyens de fixation comprennent un matériau adhésif appliqué sur la première face 2a du support 1 de sorte à maintenir le support 1 sur la peau par l'adhésif. Préférentiellement, l'adhésif est un matériau adhésif sur ces deux faces pour coopérer avec la première face 2a du support et avec la zone de la peau sur laquelle le support doit être appliqué. L'adhésif est avantageusement biocompatible, par exemple un adhésif hydrocolloïde double face.

Le support 1 comprend un circuit électrique imprimé 6 destiné à connecter les électrodes 4. Avantageusement, le circuit imprimé 6 est disposé sur la première face 2a du support 1. Préférentiellement, le circuit imprimé 6 est isolé électriquement de la peau sur laquelle le dispositif est destiné à être appliqué. A titre d'exemple une couche de matériau isolant est appliqué sur le circuit imprimé 6. La couche de matériau isolant exclu les électrodes 4. Préférentiellement, la couche de matériau isolant exclu les moyens de connexion 5.

Le support 1 comprend des moyens de connexion 5 destiné à relié le module électronique 3 au circuit imprimé 6.

Selon un mode de réalisation avantageux, le circuit imprimé 6, les électrodes 4 et les moyens de connexion 5 sont formés sur la premier face 2a du support 1. Ceci permet une fabrication aisée avec des dépôts de couches électroniques sur une seule face du support 1.

A titre d'exemple les électrodes 4, le circuit imprimé 6 et les moyens de connexion 5 sont formés par impression avec une encre conductrice sur le support 1. A titre d'exemple des encres conductrices à bases d'argent, d'or ou d'oxyde de zinc peuvent être utilisées. Préférentiellement, les électrodes 4 destinées à être en contact avec l'utilisateur sont en matériau bioconducteur tel que par exemple le PEDOT-PSS (poly(3,4-éthylènedioxythiophène) - poly(styrène sulfonate) de sodium. Le circuit électrique imprimé 6 peut également être en fibres conductrices tissés dans un textile.

Selon l'invention, le support 1 comprend avantageusement des moyens de détachement partiel 7 portant au moins une partie des moyens de connexion 5. Les moyens de détachement partiel 7 sont destinés à permettre aux moyens de connexion 5 formés sur la première face 2a du support 1 d'être déplacés au niveau de la deuxième face 2b et préférentiellement au-delà de la deuxième face 2b.

Préférentiellement, la première face 2a définit un plan dans lequel sont agencés les électrodes 4 et le circuit imprimé 6. Les moyens de connexion 5 sont configurés pour pouvoir être agencés dans le plan de la première face 2a et en dehors de celui-ci préférentiellement au-delà de la deuxième face 2b lorsque les moyens de connexion 5 sont actifs. On entend par actif que les moyens de connexion 5 sont connectés au module électronique 3. A l'inverse les moyens de connexion 5 sont dits inactifs lorsqu'ils ne sont pas connectés au module électronique 3.

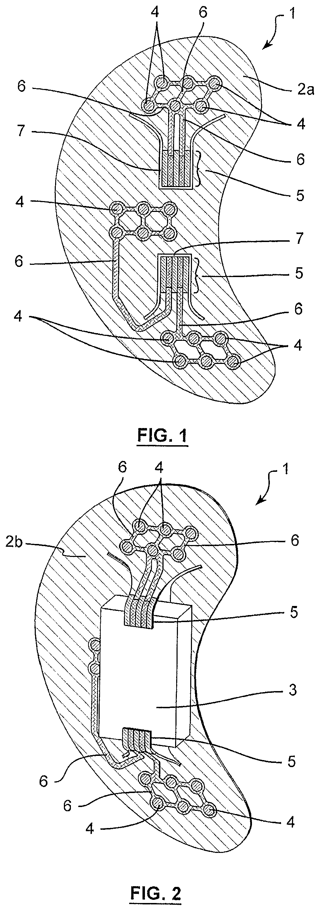

Selon un mode de réalisation, les moyens de détachement partiel 7 sont formés par des découpes du support 1 autour des moyens de connexion 5. Les moyens de détachement partiel 7 définissent au moins une languette, préférentiellement deux languettes. Chaque languette porte au moins en partie des moyens de connexion 5. Les moyens de détachement partiels 7 sont flexibles pour être fléchis et déportés en dehors de la première face 2a préférentiellement au niveau de la deuxième face 2b ou au-delà. Les découpes sont agencées par exemple telles que représentées sur les figures 1 à 3. Les moyens de détachement partiel 7, préférentiellement chaque languette comprend une zone de liaison assurant la connexion électrique entre le circuit imprimé 6 et les moyens de connexion 5. La connexion avec le circuit imprimé 6 est maintenue. L'extrémité libre des moyens de détachement partiel 7, préférentiellement opposée à la zone de liaison et donc de connexion au circuit imprimé 6 comprend avantageusement les moyens de connexion 5 destinés à être connectés au module électronique 3. Cette extrémité libre est mobile par rapport au support 1.

Cette disposition permet une fabrication sur une seule face du support 1 tout en permettant une connexion au module électronique 3 sur la face opposée.

Selon un mode de réalisation préféré de l'invention, les moyens de connexion 5 sont disposés dans le plan de la première face 2a du support 1 lorsqu'ils ne sont pas reliés au module électronique 3 et ils sont disposés en dehors du plan de la première face 2a du support 1 lorsqu'ils sont reliés au module électronique 3.

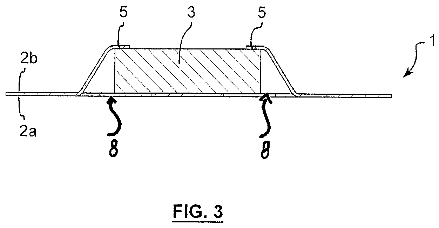

A titre préféré, les moyens de connexion 5 sont des moyens simultanément de connexions électriques et physiques. C'est-à-dire que les moyens de connexion 5 assurent une connexion électrique du module électronique 3 et du circuit imprimé 6 et simultanément ils assurent une fixation physique du module électronique 3 sur le support 1. Préférentiellement, tel que représenté en figure 3, le module électronique 3 est en appui sur la deuxième face 2b du support et les moyens de connexion 5 viennent s'appliquer sur des zones complémentaires du module électronique 3. Le module électronique 3 est disposé entre la deuxième face 2b du support 1 et au moins une languette préférentiellement les deux languettes. Préférentiellement les moyens de connexion 5 plus précisément les languettes définies par les moyens de détachement partiel 7 sont en regard l'une de l'autre de sorte à maintenir le module électronique 3. Le module électronique 3 est supporté par le support 1, c'est-à-dire que le support 1 s'oppose à la chute par gravité du module électronique 3. Avantageusement, le module électronique 3 est en appui par toute sa surface agencée en regard de la deuxième face 2b.

Le détachement des languettes au-delà de la première face 2a vers ou delà de la deuxième face 2b s'oppose à une force de rappel. De cette manière lorsque les languettes sont agencées au-dessus du module électronique 3 et que les moyens de connexion 5 coopèrent avec le module électronique 3, elles exercent une force d'appui sur le module électronique 3 de sorte à le plaquer sur la deuxième face 2b du support 1. A titre d'exemple, les languettes ont une longueur de 1 à 3 cm.

Selon un mode de réalisation avantageux, le module électronique 3 comprend des moyens de mesures de paramètres physiologiques de l'utilisateur. Préférentiellement, au moins une sonde, par exemple de mesure de la température, est agencée sur la face du module électronique 3 au regard de la deuxième face 2b du support 1 au niveau d'ouverture 8 formée par le déplacement des moyens de connexions au niveau de la deuxième face 2b du support 1 pour être connectés au module électronique 3. De cette manière, la sonde est agencée sensiblement au niveau de la première face 2a du support 1 et peut donc être en contact de la peau une fois le dispositif appliqué sur l'utilisateur.

Le module électronique 3 est avantageusement destiné à contrôler le courant fourni. Le module électronique 3 permet avantageusement de contrôler l'intensité, la tension et/ou la durée d'administration. Selon une possibilité, le module électronique 3 comprend un microprocesseur alimenté par une source d'énergie électrique telle qu'un accumulateur rechargeable ou une pile et qui commande l'application d'une tension aux électrodes 4 par exemple par l'intermédiaire d'un générateur de tension.

La source d'énergie peut être diverse. Avantageusement, la source d'énergie est autonome, à titre d'exemple un générateur autonome d'énergie par récupération d'énergie peut être utilisé ou bien des batteries de type piles.

Selon un mode de réalisation avantageux, le module électronique 3 comprend la source de courant, préférentiellement une source de courant autonome. Cette disposition est particulièrement avantageuse pour permettre un usage ambulatoire du dispositif selon l'invention.

Selon un mode de réalisation préféré, le module électronique 3 est séparable du support 1. Plus précisément, le module électronique 3 est amovible du support 1. Préférentiellement, le module électronique 3 est réutilisable. Le support 1 est lui avantageusement un consommable qui va être utilisé une seule fois ou quelque fois mais dans une mesure plus faible que le module électronique 3. Le support 1 est jetable. Ceci est particulièrement intéressant d'un point de vue écologique et économique car le support 1 comprend les électrodes 4. Or, le déposant a pu constater que les électrodes des dispositifs d'électrostimulation et/ou iontophorèse subissaient de forte oxydation et donc un endommagement au cours du temps. Cette disposition permet de jeter le support 1 après utilisation sans jeter la partie électronique qui coute plus chère. De plus on peut avec un seul module électronique 3 administrer plusieurs substances actives ou bien faire alternativement de l'électrostimulation et de l'iontophorèse uniquement en changeant de support 1.

Le dispositif selon l'invention permet d'avoir un dispositif qui puisse à la fois être à usage unique ou du moins utilisé pour un seul patient tout en réutilisant les éléments électroniques coûteux.

Selon l'invention, le support 1 pouvant également se nommer timbre est une nappe souple. La souplesse est telle que deux points opposés du pourtour peuvent être accolés, sans détérioration du support 1. Cette souplesse est avantageusement maintenue lorsque le module électronique 3 est connecté et fixé sur le support 1. Préférentiellement, le support 1 présente une épaisseur de 40 à 60 µm. La souplesse du support 1 permet au dispositif de se conformer à la zone d'application du corps où il est destiné à être appliqué.

Le dispositif selon l'invention est utilisable sur l'ensemble des zones du corps humain facilement et sans gêne ou désagrément pour l'utilisateur.

Le support 1 présente une surface supérieure à celle du module électronique 3. Plus précisément, la surface de la première face 2a du support 1 est plus grande que la surface du module électronique 3 en regard du support 1. Avantageusement, le ratio des surfaces première face 2a / module électronique 3 est au moins égale à 1/5. Cette différence de taille permet notamment de maintenir une souplesse du support pour une bonne application sur toute la zone à traiter.

Préférentiellement, le module électronique 3 à une surface en regard du support 1 de l'ordre de 1 à 6 cm², préférentiellement la première face 2a du support 1 est de l'ordre de 8 à 50 cm².

A titre d'exemple, le support 1 a une épaisseur de 50µm et des dimensions de 41 mm de largueur pour 142 mm de longueur.

La surface du support 1 est avantageusement adaptable à la surface sur laquelle il est destiné à être appliqué. A titre d'exemple, le support 1 comprend une portion centrale et de part et d'autre de la portion centrale s'étend au moins une portion élargie portant au moins deux électrodes. Avantageusement, le support 1 est sécable pour s'adapter à la surface sur laquelle il est destiné à être appliqué. Dans un mode de réalisation dans lequel le support 1 comprend deux portions élargies s'étendant de part et d'autres de la portion centrale, le support 1 peut être sectionné entre deux portions élargies pour réduire la surface d'application du support 1. La souplesse du support 1 permet avantageusement cette coupure. On entend par sécable que le support 1 est configuré pour être découpé, sectionné et ceci même au niveau du circuit électrique 6.

Le dispositif selon l'invention peut être utilisé dans des applications cosmétiques ou thérapeutiques. Par exemple, si le dispositif est utilisé pour l'électrostimulation, le courant pénètre dans la peau pour, soit stimuler les fibroblastes de manière à augmenter leur taux de production de collagène et d'élastine dont l'effet est une amélioration de l'élasticité de la peau, soit traiter une cicatrisation. Si le dispositif est utilisé pour l'iontophorèse, il destiné à faire pénétrer des substances actives dans le derme, telles que par exemple la vitamine A ou rétinol ayant un effet dépigmentant, l'acide rétinoïque pour le traitement de l'acné, la vitamine C ayant un effet antioxydant, un chélateur d'ions tel que l'acide β-alanine di-acétique pour le traitement des érythèmes, l'acide glycolique améliorant la texture de la peau, le phosphate sodique de dexaméthasone ayant un effet anti-inflammatoire ou tout autre type d'actif ionisé ou présenté sous forme d'émulsions anioniques ou cationiques afin de pouvoir être véhiculé par le courant.

Le module électronique 3 est configuré pour délivrer un courant de faible tension et de densité limitée. A titre préféré, la tension U est inférieure ou égale à 1 V et l'intensité I est inférieure ou égale à 1 mA.

Préférentiellement, le support 1 comprend un matériau plastique. A titre d'exemple, le support 1 est en polyimide (PI), polyéthylène téréphtalate (PET), polyéther éther cétone (PEEK), polyéthylène naphthalate (PEN).

Selon un autre mode de réalisation, le support 1 comprend une trame textile par exemple du nylon.

Le matériau du support 1 est préférentiellement fin. Le matériau du support 1 est préférentiellement résistant à des températures élevées pour des éventuels recuits de l'encre conductrice.

Le support 1 est appliqué seul pour de l'électrostimulation ou avec une couche intercalaire pour l'iontophorèse.

Selon un mode de réalisation, le dispositif comprend de la biocellulose préférentiellement imbibée par exemple de substances actives pour l'iontophorèse.

Selon un autre mode de réalisation, le dispositif comprend un gel contenant de substances actives pour l'iontophorèse. Le gel peut être appliqué directement sur la peau puis le support 1 est appliqué par-dessus ou bien le gel est appliqué sur le support 1 puis l'ensemble est ensuite appliqué sur la peau. Le gel peut former les moyens de fixations du support 1 sur la peau notamment en présentant des propriétés d'adhésion.

A titre d'exemple non limitatif, les moyens de fixation type adhésif et/ou la couche intercalaire contenant des substances actives mesurent de 25 à 60 µm d'épaisseur.

Le module électronique 3 comprend selon des possibilités, des capteurs des paramètres physiologiques tels que la température, le pH, la résistivité et/ou des témoins lumineux tels que des LEDs (diodes électroluminescentes)

Selon une possibilité avantageuse, le dispositif comprend une pluralité d'anodes et une pluralité de cathodes alternées avec les anodes. Dans cette configuration, le courant total se divise suivant plusieurs circuits anode-cathode de manière à ce que la densité du courant circulant d'une anode à une cathode soit préférentiellement inférieure à 1mA/cm2. En outre, afin d'empêcher la formation de lignes de champ externes, c'est-à-dire à l'extérieur du dispositif, les deux électrodes se trouvant aux deux extrémités sont de même polarité, c'est à dire que ce sont deux anodes ou deux cathodes.

### REFERENCES

- 1: Support
- 2a.: Première face
- 2b.: Deuxième face
- 3: Module électronique
- 4: Electrodes
- 5: Moyens de connexion
- 6: Circuit électrique imprimé
- 7: Moyens de détachement partiel
- 8: Ouverture

## Revendications

1. Dispositif pour l'électrostimulation et/ou l'iontophorèse comprenant un support (1) présentant une première face (2a) destinée à être placée au regard de la peau et portant des électrodes (4) configurées pour être au contact de la peau et une deuxième face (2b) opposée à la première sur laquelle est destiné à être disposé un module électronique (3) **caractérisé par le fait que**
le module électronique (3) est configuré pour être amovible du support (1) et que le support (1) comprend sur sa première face (2a) un circuit électrique (6) reliant les électrodes (4) et des moyens de connexion (5) destinés à connecter le circuit électrique (6) au module électronique (3) et que le support (1) comprend des moyens de détachement partiel (7) flexibles formant au moins une languette portant les moyens de connexion (5) et configurés pour permettre de rabattre par flexion la au moins une languette pour amener lesdits moyens de connexion (5) au-delà de la première face (2a) au niveau ou au-delà de la deuxième face (2b) pour connecter le module électronique (3), le module électronique (3) étant en appui sur la deuxième face (2b) du support (1) et disposé entre ladite deuxième face (2b) du support (1) et la au moins une languette.

2. Dispositif selon la revendication précédente dans lequel le module électronique (3) est configuré pour être séparable du support (1).

3. Dispositif selon l'une quelconque des revendications précédentes dans lequel les moyens de détachement partiel (7) forment au moins deux languettes.

4. Dispositif selon la revendication précédente dans lequel les deux languettes rabattues par flexion au-delà de la première face (2b) au niveau ou au-delà de la deuxième face (2b) pour connecter les moyens de connexion (5) au module électronique (3) exercent une force d'appui sur ledit module électronique (3) configurée pour plaquer ledit module électronique (3) sur la deuxième face (2b) du support (1).

5. Dispositif selon la revendication précédente dans lequel le module électronique (3) est entièrement soutenu par la deuxième face (2b) du support (1).

6. Dispositif selon l'une quelconque des revendications précédentes dans lequel les moyens de connexion (5) et le module électronique (3) sont configurés pour coopérer de manière à ce que leur coopération assure simultanément une connexion électrique du module électronique (3) au circuit électrique (6) et un maintien physique du module électronique (3) sur la deuxième face (2b) du support (1).

7. Dispositif selon l'une quelconque des revendications précédentes dans lequel les moyens de connexion (5) et le module électronique (3) sont configurés pour coopérer de manière à ce que leur coopération assure à elle seule simultanément une connexion électrique du module électronique (3) au circuit électrique (6) et un maintien physique du module électronique (3) sur la deuxième face (2b) du support (1).

8. Dispositif selon la revendication précédente dans lequel le maintien physique du module électronique (3) est uniquement assuré par les moyens de connexion (5) et le support (1).

9. Dispositif selon l'une quelconque des revendications précédentes dans lequel le support (1) est une nappe souple de surface supérieure à celle du module électronique (3) configurée pour se conformer aux zones d'application sur le corps humain sur lesquelles elle est destinée à être appliquer, préférentiellement la surface du support (1) est au moins cinq fois supérieure à celle du module électronique (3).

10. Dispositif selon l'une quelconque des revendications précédentes dans lequel le support (1) est sécable de sorte à être adaptable à la surface sur laquelle il est destiné à être appliqué.

11. Dispositif selon l'une quelconque des revendications précédentes comprenant un film de matériau isolant électriquement destiné à isoler électriquement ledit circuit électrique (6) de la peau sur laquelle le dispositif est destiné à être appliqué.

12. Dispositif selon l'une quelconque des revendications précédentes dans lequel le circuit électrique (6), les électrodes (4) et les moyens de connexion (5) sont formés par une encre conductrice imprimée ou sérigraphiée sur la première face (2a) du support (1).

13. Dispositif selon l'une quelconque des revendications précédentes dans lequel le support (1) comprend au moins un élément actif cosmétique ou thérapeutique disposé sur sa première face (2a), préférentiellement le support (1) comprend une trame en biocellulose imbibé sur la première face (2a) du support (1) d'au moins un élément actif ou le support (1) comprend une trame textile recouverte sur la première face (2a) du support (1) d'un hydrogel contenant au moins un élément actif.

14. Dispositif selon l'une quelconque des revendications précédentes dans lequel le support (1) comprend un adhésif sur sa première face (2a) de sorte à maintenir le dispositif appliqué sur la peau.

15. Dispositif selon l'une quelconque des revendications précédentes dans lequel le module électronique (3) comprend une source de courant autonome et le support (1) est à usage unique.

16. Procédé de fabrication d'un dispositif d'électrostimulation et/ou d'iontophorèse suivant l'une quelconque des revendications précédentes comprenant les étapes suivantes : réalisation par dépôt d'au moins une couche de matériau conducteur sur une première face (2a) d'un support (1) d'électrodes (4), d'un circuit électrique (6) reliant les électrodes (4) et de moyens de connexion (5), dépôt d'une couche d'un matériau isolant sur le circuit électrique (6) de sorte à l'isoler électriquement de la peau sur laquelle le dispositif est destiné à être appliqué, formation par découpe des moyens de détachement partiel (7) des moyens de connexion (5).

17. Procédé d'assemblage d'un dispositif selon suivant l'une quelconque des revendications précédentes comprenant la flexion des moyens de détachement partiel (7) de sorte à amener les moyens de connexion (5) au niveau ou delà de la deuxième face (2b) du support (1) et la connexion desdits moyens de connexion (5) avec le module électronique (3).

## Patentansprüche

1. Vorrichtung zur Elektrostimulation und/oder lontophorese, umfassend einen Träger (1), der eine erste Fläche (2a) aufweist, die dazu vorgesehen ist, der Haut zugewandt platziert zu werden, und die Elektroden (4) trägt, die dafür ausgebildet sind, mit der Haut in Kontakt zu stehen, und eine der ersten gegenüberliegende zweite Fläche (2b), auf der ein elektronisches Modul (3) vorgesehen ist, angeordnet zu werden,
**dadurch gekennzeichnet, dass**
das elektronische Modul (3) dafür ausgebildet ist, vom Träger (1) abnehmbar zu sein, und dass der Träger (1) auf seiner ersten Fläche (2a) einen elektrischen Schaltkreis (6) umfasst, der die Elektroden (4) verbindet, und Anschlussmittel (5), die dazu vorgesehen sind, den elektrischen Schaltkreis (6) an das elektronische Modul (3) anzuschließen, und dass der Träger (1) biegsame Mittel zum teilweisen Ablösen (7) umfasst, die mindestens eine Lasche bilden, die die Anschlussmittel (5) trägt und dafür ausgebildet sind, das Umschlagen durch Biegen der mindestens einen Lasche zu ermöglichen, um die Anschlussmittel (5) über die erste Fläche (2a) hinaus auf Ebene der oder über die zweite Fläche (2b) hinaus zu bringen, um das elektronische Modul (3) anzuschließen, wobei das elektronische Modul (3) auf der zweiten Fläche (2b) des Trägers (1) aufliegt und zwischen der zweiten Fläche (2b) des Trägers (1) und der mindestens einen Lasche angeordnet ist.

2. Vorrichtung nach dem vorstehenden Anspruch, wobei das elektronische Modul (3) dafür ausgebildet ist, vom Träger (1) trennbar zu sein.

3. Vorrichtung nach einem der vorstehenden Ansprüche, wobei die Mittel zum teilweisen Ablösen (7) mindestens zwei Laschen bilden.

4. Vorrichtung nach dem vorstehenden Anspruch, wobei die zwei Laschen, die durch Biegen über die erste Fläche (2b) hinaus auf Ebene der oder über die zweite Fläche (2b) hinaus umgeschlagenen sind, um die Anschlussmittel (5) an das elektronische Modul (3) anzuschließen, eine Auflagekraft auf das elektronische Modul (3) ausüben, die dafür ausgebildet ist, das elektronische Modul (3) auf die zweite Fläche (2b) des Trägers (1) zu drücken.

5. Vorrichtung nach dem vorstehenden Anspruch, wobei das elektronische Modul (3) vollständig von der zweiten Fläche (2b) des Trägers (1) gestützt wird.

6. Vorrichtung nach einem der vorstehenden Ansprüche, wobei die Anschlussmittel (5) und das elektronische Modul (3) dafür ausgebildet sind, derart zusammenzuwirken, dass ihr Zusammenwirken gleichzeitig einen elektrischen Anschluss des elektronischen Moduls (3) an den elektrischen Schaltkreis (6) und einen physikalischen Halt des elektronischen Moduls (3) auf der zweiten Fläche (2b) des Trägers (1) sicherstellt.

7. Vorrichtung nach einem der vorstehenden Ansprüche, wobei die Anschlussmittel (5) und das elektronische Modul (3) dafür ausgebildet sind, derart zusammenzuwirken, dass alleine ihr Zusammenwirken gleichzeitig einen elektrischen Anschluss des elektronischen Moduls (3) an den elektrischen Schaltkreis (6) und einen physikalischen Halt des elektronischen Moduls (3) auf der zweiten Fläche (2b) des Trägers (1) sicherstellt.

8. Vorrichtung nach dem vorstehenden Anspruch, wobei der physikalische Halt des elektronischen Moduls (3) ausschließlich von den Anschlussmitteln (5) und dem Träger (1) sichergestellt wird.

9. Vorrichtung nach einem der vorstehenden Ansprüche, wobei der Träger (1) eine weiche Bahn von größerer Oberfläche als derjenigen des elektronischen Moduls (3) ist, die dafür ausgebildet ist, sich an die Anwendungsbereiche am menschlichen Körper anzuschmiegen, auf denen sie vorgesehen ist, angewendet zu werden, wobei die Oberfläche des Trägers (1) vorzugsweise mindestens fünfmal größer ist als diejenige des elektronischen Moduls (3).

10. Vorrichtung nach einem der vorstehenden Ansprüche, wobei der Träger (1) schneidbar ist, sodass er an die Oberfläche, auf der er vorgesehen ist, angewendet zu werden, anpassbar ist.

11. Vorrichtung nach einem der vorstehenden Ansprüche, umfassend einen Film aus elektrisch isolierendem Material, der dazu vorgesehen ist, den elektrischen Schaltkreis (6) elektrisch von der Haut zu isolieren, auf der die Vorrichtung vorgesehen ist, angewendet zu werden.

12. Vorrichtung nach einem der vorstehenden Ansprüche, wobei der elektrische Schaltkreis (6), die Elektroden (4) und die Anschlussmittel (5) von einer leitfähigen Tinte gebildet werden, die auf der ersten Fläche (2a) des Trägers (1) aufgedruckt oder siebgedruckt ist.

13. Vorrichtung nach einem der vorstehenden Ansprüche, wobei der Träger (1) mindestens ein kosmetisch oder therapeutisch wirksames Element umfasst, das auf seiner ersten Fläche (2a) angeordnet ist, der Träger (1) vorzugsweise einen Schussfaden aus Bio-Cellulose umfasst, die auf der ersten Fläche (2a) des Trägers (1) mit mindestens einem wirksamen Element getränkt ist, oder der Träger (1) einen textilen Schussfaden umfasst, der auf der ersten Fläche (2a) des Trägers (1) mit einem Hydrogel bedeckt ist, das mindestens ein aktives Element enthält.

14. Vorrichtung nach einem der vorstehenden Ansprüche, wobei der Träger (1) auf seiner ersten Fläche (2a) einen Klebstoff umfasst, sodass er die angewendete Vorrichtung auf der Haut hält.

15. Vorrichtung nach einem der vorstehenden Ansprüche, wobei das elektronische Modul (3) eine autonome Stromquelle umfasst und der Träger (1) zum einmaligen Gebrauch ist.

16. Verfahren zum Herstellen einer Elektrostimulations- und/oder lontophoresevorrichtung nach einem der vorstehenden Ansprüche, umfassend die folgenden Schritte: Ausführen, durch Abscheiden von mindestens einer Schicht aus leitendem Material auf einer ersten Fläche (2a) eines Trägers (1), von Elektroden (4), von einem elektrischen Schaltkreis (6), der die Elektroden (4) verbindet, und von Anschlussmitteln (5), Abscheiden einer Schicht aus einem isolierenden Material auf dem elektrischen Schaltkreis (6), sodass er elektrisch von der Haut isoliert wird, auf der die Vorrichtung vorgesehen ist, angewendet zu werden, Bilden, durch Ausschneiden, von Mitteln zum teilweisen Ablösen (7) der Anschlussmittel (5).

17. Verfahren zum Zusammenbauen einer Vorrichtung nach einem der vorstehenden Ansprüche, umfassend das Biegen der Mittel zum teilweisen Ablösen (7), sodass die Anschlussmittel (5) auf Ebene der oder über die zweite Fläche (2b) des Trägers (1) hinaus gebracht werden, und das Anschließen der Anschlussmittel (5) an das elektronische Modul (3).

## Claims

1. Device for electrostimulation and/or iontophoresis comprising a mounting (1) having a first face (2a) intended to be placed facing the skin and supporting electrodes (4) configured to be in contact with the skin and a second face (2b) opposite the first on which is intended to be arranged an electronic module (3) **characterised by** the fact that the electronic module (3) is configured to be detachable from the mounting (1) and that the mounting (1) comprises on its first face (2a) an electric circuit (6) connecting the electrodes (4) and connection means (5) intended to connect the electric circuit (6) to the electronic module (3) and that the mounting (1) comprises flexible means for partial detachment (7) forming at least one tab supporting the connection means (5) and configured to make it possible to fold back by bending the at least one tab in order to bring said connection means (5) beyond the first face (2a) on or beyond the second face (2b) in order to connect the electronic module (3), with the electronic module (3) bearing against the second face (2b) of the mounting (1) and arranged between said second face (2b) of the mounting (1) and the at least one tab.

2. Device according to the preceding claim wherein the electronic module (3) is configured to be able to be separated from the mounting (1).

3. Device according to any preceding claim wherein the means for partial detachment (7) form at least two tabs.

4. Device according to the preceding claim wherein the two tabs folded back by bending beyond the first face (2b) on or beyond the second face (2b) in order to connect the connection means (5) to the electronic module (3) exert a support force on said electronic module (3) configured to thrust said electronic module (3) on the second face (2b) of the mounting (1).

5. Device according to the preceding claim wherein the electronic module (3) is entirely supported by the second face (2b) of the mounting (1).

6. Device according to any preceding claim wherein the connection means (5) and the electronic module (3) are configured to cooperate in such a way that their cooperation simultaneously provides an electric connection from the electronic module (3) to the electric circuit (6) and a physical maintaining of the electronic module (3) on the second face (2b) of the mounting (1).

7. Device according to any preceding claim wherein the connection means (5) and the electronic module (3) are configured to cooperate in such a way that their cooperation provides in itself simultaneously an electric connection from the electronic module (3) to the electric circuit (6) and a physical maintaining of the electronic module (3) on the second face (2b) of the mounting (1).

8. Device according to the preceding claim wherein the physical maintaining of the electronic module (3) is solely provided by the connection means (5) and the mounting (1).

9. Device according to any preceding claim wherein the mounting (1) is a flexible layer with a surface greater than that of the electronic module (3) configured to conform to the zones of application on the human body whereon it is intended to be applied, preferably the surface of the mounting (1) is at least five times greater than that of the electronic module (3).

10. Device according to any preceding claim wherein the mounting (1) can be scored in such a way as to be adapted to the surface whereon it is intended to be applied.

11. Device according to any preceding claim comprising a film of electrically insulating material intended to electrically insulate said electric circuit (6) from the skin whereon the device is intended to be applied.

12. Device according to any preceding claim wherein the electric circuit (6), the electrodes (4) and the connection means (5) are formed by a printed or silk-screened conductive ink on the first face (2a) of the mounting (1).

13. Device according to any preceding claim wherein the mounting (1) comprises at least one active cosmetic or therapeutic element arranged on its first face (2a), preferably the mounting (1) comprises a soaked biocellulose weave on the first face (2a) of the mounting (1) of at least one active element or the mounting (1) comprises a textile weave covered on the first face (2a) of the mounting (1) with a hydrogel containing at least one active element.

14. Device according to any preceding claim wherein the mounting (1) comprises an adhesive on its first face (2a) in such a way as to maintain the device applied on the skin.

15. Device according to any preceding claim wherein the electronic module (3) comprises an autonomous source of current and the mounting (1) is for single use.

16. Method for manufacturing a device for electrostimulation and/or iontophoresis according to any of the preceding claims comprising the following steps: realising via deposition of at least one layer of conductive material on a first face (2a) of a mounting (1) of electrodes (4), of an electric circuit (6) connecting the electrodes (4) and connection means (5), deposition of a layer of insulating material on the electric circuit (6) in such a way as to electrically insulate the skin whereon the device is intended to be applied, forming via cut-out of the means for partial detachment (7) of the connection means (5).

17. Method for assembling a device according to any of the preceding claims comprising the bending of the means for partial detachment (7) in such a way as to bring the connection means (5) on or beyond the second face (2b) of the mounting (1) and the connecting of said connection means (5) with the electronic module (3).
